# EUROPEAN PATENT SPECIFICATION

(11) **EP 1 341 449 B1**
(45) Date of publication and mention of the grant of the patent: **14.12.2005**
(21) Application number: 01270279.1
(22) Date of filing: 21.11.2001
(51) Int. Cl.: A61B 17/12

(54) **HEMOSTATIC DEVICE**
HÄMOSTASEGERÄT
DISPOSITIF HEMOSTATIQUE

(30) Priority: 16.12.2000 GB 0030794
(43) Date of publication of application: 10.09.2003
(73) Proprietor: BHK Holding, Grand Cayman (KY)
(72) Inventor: Hudson, John Overton, Glenfield, Leicester LE3 8AG (GB); Bauer, Alberto, 29600 Marbella/Malaga (ES)
(74) Representative: Kazi, Ilya
(86) International application number: PCT/GB2001/005116
(87) International publication number: WO 2002/047558

(56) References cited:
- EP-A- 0 864 301
- WO-A-00/48517
- WO-A-00/72909
- WO-A-01/23653
- WO-A-98/57586
- US-A- 2 847 997
- US-A- 4 950 280

## Description

### Field of the Invention

The present invention relates to haemostatic devices for reducing bleeding in body cavities and vessels. These devices also include use in post operative nasal packing and similar uses in other body cavities.

### Background of the Invention

Haemostatic devices are known for the treatment of bleeding inside a body cavities and vessels. Their aim of their use is the prevention of blood flow, that is, haemostasis and for post operative packing to control bleeding and promote healing. Various haemostatic and packing devices are known including sponge materials which expands when wetted and balloon devices which can be expanded hydraulically or pneumatically, both of which can apply pressure to the source of the bleeding.

### Sponges

Sponges typically consist of a polymeric material such as polyvinyl alcohol (PVA) which can be compressed when dry and which expand when wetted. When a dry sponge is introduced into a bleeding cavity it can expand by absorbing blood and other fluids. The expansion of the sponge can apply light pressure to the source of the bleeding.

Such devices, whilst somewhat effective in reducing or stopping bleeding have a number of disadvantages. They are hard and uncomfortable to the patient, being very painful during deployment. They are not able to supply sufficient pressure to the lesion. They do not have inherent haemostatic properties, but work by soaking up the excess blood until it forms a clot inside and around the sponge.

Thus, when used as a haemostatic device, the sponge is incorporated into the formed blood clot. This dries hard and so the device is difficult to remove without causing damage to the blood clot and subsequent re-bleeding. Removal of the sponge can therefore be a painful and upsetting experience for the patient.

Furthermore, expansion of the sponge blocks the cavity or vessel. This can be undesirable for instance when being used in the treatment of epistaxis (intra nasal bleeding) in the nasal cavity because this can prohibit breathing via the nasal passage. Modified sponges are available which incorporate a breathing tube but nevertheless such devices remain inefficient and uncomfortable for the patient In addition, because known sponges have no actual haemostatic properties, they require considerable periods of time (up to 48 hours) in position before they become effective in reducing bleeding. This may cause other problems such as toxic shock syndrome.

### Balloon Devices

Balloon devices typically consist of a balloon mounted on a tubular catheter. The balloon is inserted into a bleeding body cavity (such as a nasal cavity) and inflated. Inflation causes the balloon to press against the source of bleeding and assists in blood clotting in order to create haemostasis by direct tamponade.

This device can be improved by covering the balloon with a haemostatic agent, such as a tubular knitted fabric manufactured from carboxymethylatedcellulose (CMC) and reinforced with nylon.

When in contact with blood or other fluids such as mucus, CMC swells and turns into a gel. The gel is a strong haemostatic agent. The nylon reinforcement maintains the integrity of the fabric after the gelling takes place, for example see International Patent Application No. PCT/GB00/03586.

This principle is used in the "Rapid Rhino™" device made by BHK Holdings of West Bay Road, PO Box 31106, SMB Grand Cayman, Cayman Islands, British West Indies which is used to treat nose bleeding. A similar device based on the same principle is designed to treat bleeding in diverticula in the colon.

However, there is some concern when using these devices in that the pressure within the balloon must be carefully controlled in order to prevent trauma. Whilst the pressure must be high enough to control the bleeding, unsuitably high pressure increases the danger of serious damage to the body cavity or vessel or "toxic shock syndrome". For example, when used as a nasal device, great care must be taken not to damage the sensitive mucus tissue in contact with the device. This risk may cause physicians to avoid using balloon devices inside the nasal cavity, even though the efficacy of the haemostatic fabric covered balloon device is well proven.

There is a need therefore for a new type of haemostatic device which:
- will apply a haemostatic fabric or other haemostatic medium to the walls of a cavity at a sustained pressure high enough to facilitate haemostasis but;
- will be incapable of exerting a dangerously high pressure;
- will be self expanding into the cavity without any external aid such as a balloon;
- remains pliable in use and "non stick" so that the removal of the device is simple with no traumatic effect;
- will, preferably, leave an airway (in the case of a nasal cavity);
- can be folded into a very small profile so that insertion is easily tolerated by the patient;
- is lightweight and more comfortable for the patient than balloon devices; and
- may be used, in selected format, for post operative packing.

### Summary of the Invention

Against this background the inventors have found that a haemostatic knitted fabric can be applied with a sustained and appropriate pressure to the inner wall of a body cavity or vessel without the use of a balloon device. Textile materials other than knitted fabric may also be used, but a knitted fabric has the properties of elasticity and softness which make it the preferred material. This can be achieved by the use of a mechanical device which is neither hydraulic nor pneumatic and keeps the fabric pressed against the wall of the cavity. Using this device the inventors have demonstrated that a knitted fabric comprising a gellable haemostatic material, such as CMC, with a reinforcing component (as described in patent application PCT/GB00/03586) can be used efficaciously to create haemostasis without the risk of exerting a dangerously high pressure on the cavity inner wall. Thus the inventors have provided a means of haemostasis that is safer than a balloon device and quicker and more efficacious than a sponge only device. Thus the invention provides a haemostatic device that does not stick to the formed blood clot and thus does not cause damage to the blood clot and subsequent re-bleeding upon removal from the cavity. Accordingly the invention describes a haemostatic device that is more comfortable to wear and less painful to remove for the patient than the prior art devices.

Accordingly the invention provides a haemostatic device suitable for use in a body cavity or vessel as specified in claim 1.

WO-A-9 857 586 forms the basis for the preamble of claim 1.

### Brief Description of the Drawings

**Fig 1-** Shows a typical fabric construction for the haemostatic fabric.
**Fig 2** - Shows a sponge covered by the fabric and anti friction liner in an expanded state.

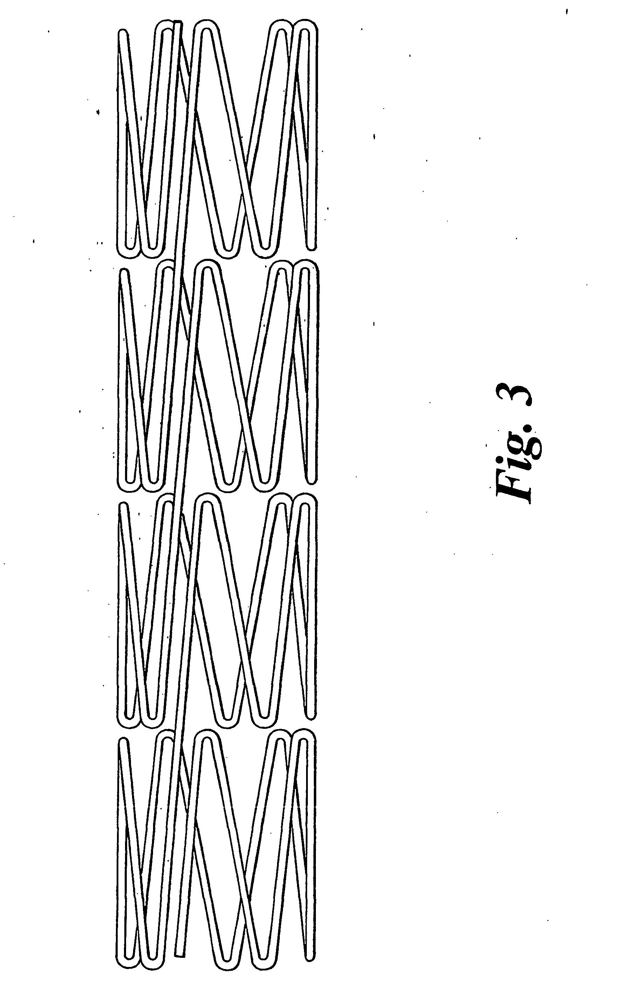

**Fig 3** - Shows the construction of a typical cylindrical, self expanding stent

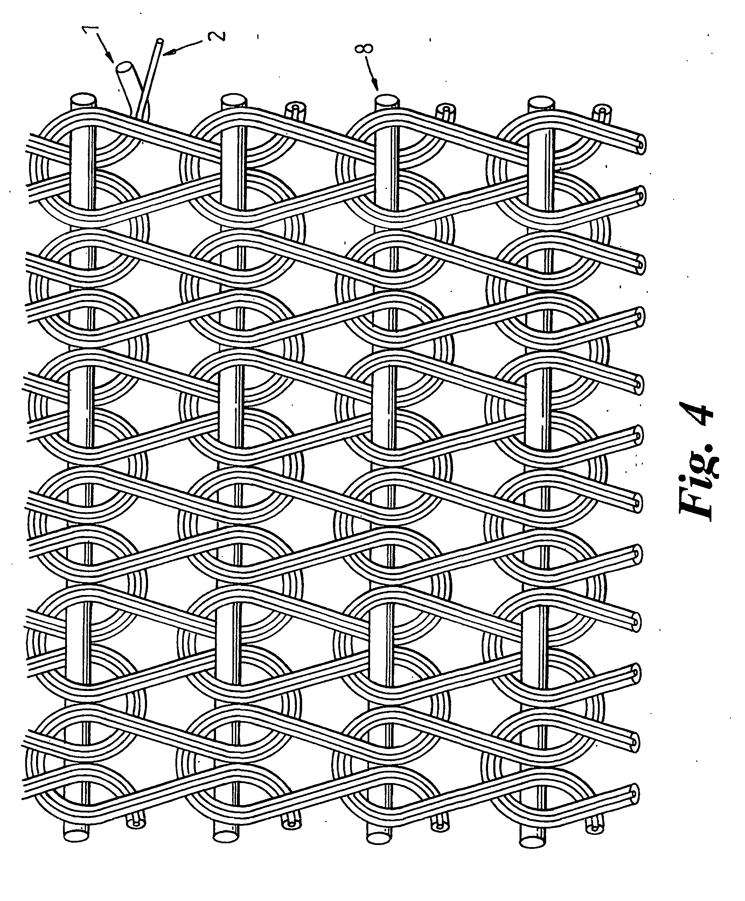

**Fig 4** - Shows a typical construction of a fabric which incorporates thermoplastic spring elements.
**Fig 5** - Shows a delivery system with a device mounted inside a thin walled tube.
**Fig 6** - Shows another knitted fabric embodiment that may be used in the construction of haemostatic devices of the present invention.

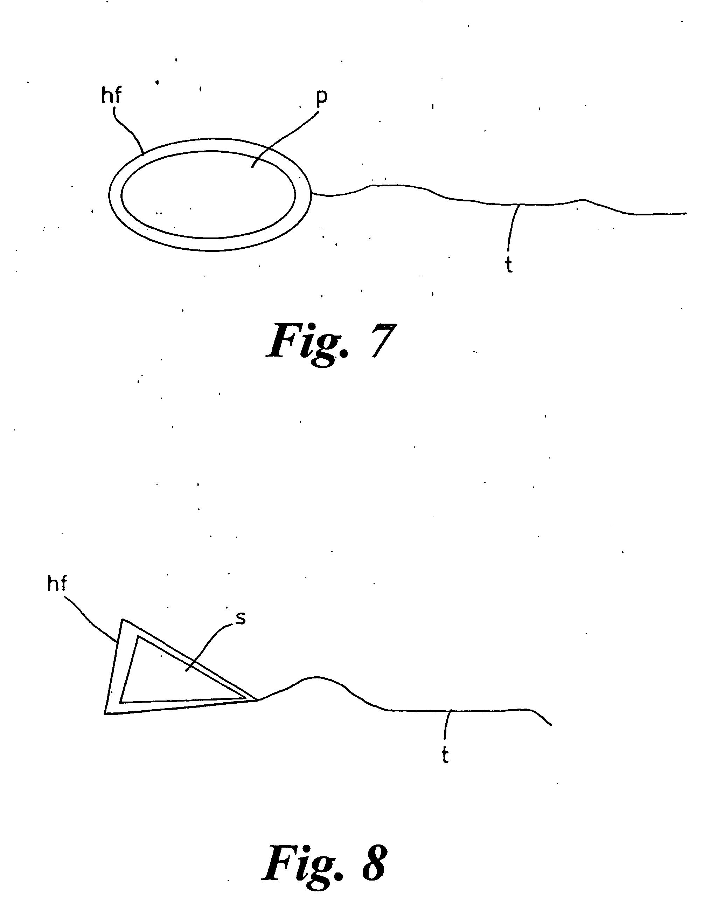

**Fig 7** - Shows an embodiment of the invention in the form of an air-filled pillow (p) covered by an hemostatic fabric (hf) and provided with a retrieval tape or string (t).
**Fig 8** - Shows another embodiment in which a substantially triangular sponge(s) is provided as the mechanical means.

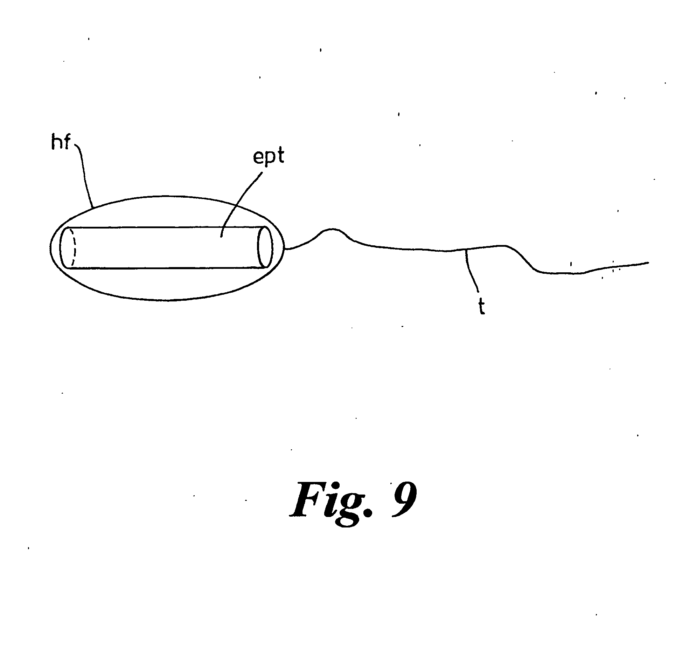

**Fig 9** - Shows a further embodiment in which an elastic plastic tube (ept) constitutes the mechanical means for expanding the hemostatic fabric (hf) outwardly and in use, against the inner wall of a cavity or vessel.

### Detailed Description of the Invention

### Haemostatic Devices

Devices of the invention can be employed in respect of most cavities or vessels of the body. In a preferred embodiment the body cavities or vessels are of the human body. Body cavities typically suitable for the present invention may include the nasal cavity, the ear, the vagina, the oesophagus, the trachea or parts of the gastric system. Preferred devices, methods and uses of the invention relate to the packing of nasal cavities. In particular, to the packing and supporting of nasal cavities after surgical procedures performed on the nose. For example, the devices, methods and uses of the invention can be employed following certain plastic surgical procedures, such as rhinoplasty and septoplasty, in which it may be necessary to cut and modify the nasal septum, that is, the cartilage-like material which separates the left and right chambers of the nose. For example the devices, methods and uses of the invention may be used in the packing of one or both nasal cavities.

Devices of the invention are suitable for use as a haemostatic device in a body cavity or vessel. The term "suitable for use in a body cavity or vessel" refers to the ability of a device to be inserted into the cavity or vessel and to bring about haemostasis.

A device is suitable for insertion if it can be provided in a form that allows insertion without substantially stretching the cavity or vessel in a manner that causes unacceptable damage, as defined below. Preferably the width of the device when provided in a compressed of folded form for insertion will be less than the width of the cavity or vessel, at least in respect of the entry point to the cavity or vessel and/or the route which the device takes through the cavity or vessel to its point of use.

Haemostasis is the condition where no bleeding occurs and so a haemostatic device should achieve this, or at least minimise the bleeding. Devices of the invention should be capable of achieving haemostatis within 48 hours. Typically a device of the invention will achieve haemostatis within 36 hours, more typically within 24 hours, 18 hours or 12 hours. Preferably a device of the invention will be capable of achieving haemostatis within 6 hours, more preferably within 4 hours, yet more preferably within 2 hours, even more preferably within 1 hour, most preferably within 30 minutes.

Typically a haemostatic device of the invention comprises a mechanical means for expanding the device against the inner surface of the cavity or vessel and a haemostatic fabric on the surface of the device.

### Mechanical Expansion Means

Devices of the invention comprise mechanical means for outwardly expanding the device such that it is capable of contacting with and placing pressure against the inner wall of a cavity or vessel into which the device can be inserted. The term "mechanical means" as used herein refers to any type of means which has an original form (relaxed state) which can be compressed and retained in a compressed state for a period of time, but which upon release will substantially return to its relaxed state. The relaxed state of the mechanical means is typically wider than the lumen of the cavity or vessel for which the device is intended. Thus if a device of the invention is positioned in a cavity or vessel and the mechanical means allowed to attempt to return to its relaxed state it will expand until either it contacts the inner surface of the cavity or vessel or it reaches its relaxed state. The expanding mechanical means can therefore cause the haemostatic fabric on the surface of the device to be applied under pressure to the inner surface of the cavity or vessel. The mechanical means used in devices of the invention do not include balloon devices.

In one embodiment the relaxed state of the mechanical means is sufficiently larger than the lumen of the cavity or vessel such that the expanded device will apply the haemostatic fabric to the inner surface of the cavity or vessel at a haemostatically effective pressure. A haemostatically effective pressure is a pressure at which the bleeding can be stopped without causing unacceptable mechanical damage to the cells and tissues of the inner surface of the cavity or vessel. The amount of acceptable damage will be apparent to the skilled person and does not, for example, include the tearing of tissue lining the inner surface of the cavity or vessel, nor does it include the haemorrhagic damage of tissues proximal to the point of contact between the device and the inner surface of the cavity or vessel such as by rupture of blood vessels underlying the contact point. Generally the suitable pressure in order to achieve haemostasis solely by tamponade is just above the ambient blood pressure. However, the devices of the invention will be effective at lower pressures than this due to the haemostatic effects of the haemostatic fabric. Thus, the pressures exerted by the devices of this invention can be below the average blood pressure which is usually quoted as 120 mm of mercury or 16 kilo pascals, typically at most 90%, 70%, 50%, 30% or less of the ambient blood pressure.

In one embodiment the mechanical means is able to be returned to the compressed state prior to removal from the cavity. This facility eases the removal of the device and improves patient comfort, This is particularly beneficial where the device is used in the nasal cavity.

In one preferred embodiment the mechanical means is a sponge and a device of the invention comprising a sponge, as a mechanical means, may be referred to as a 'sponge device'. In another preferred embodiment the mechanical means is a stent and a device of the invention comprising a stent, as a mechanical means, may be referred to as a 'stent device'.

The term "sponge" as used herein refers to a polymeric material which is capable of being compressed from its relaxed state, being held in the compressed state by a restraining means and expanding to substantially the same relaxed state upon removal of said restraining means. In one form, the sponge material used in sponge devices of the invention may be compressed and dried in the compressed state. Such devices are referred to herein as 'dry sponge' devices. A dry sponge device, when compressed and dried will maintain the compressed state as long as it remains dry. Rehydrating the sponge releases the restraint and allows the sponge to return to its original, uncompressed state thus expanding the device. A preferred material for the dry sponge device of the invention is polyvinylalcohol (PVA). However, the invention contemplates the use of other sponge mechanical means which may be formed from any material that is suitable for the role described herein and, particularly in the case of dry sponge devices, typically include a sponge materials that can be considered functional equivalents of polyvinyl alcohol. Typically sponges can be compressed and restrained by a mechanical means, and then expanding to the original state after the release of the restraint. Preferably a sponge device of the invention comprises tube which passes through the device and is capable of allowing the passage of gases, such as to allow breathing by the patient when the device is used in the nasal cavity.

In one embodiment a sponge device of the invention may be prepared for insertion into a cavity or vessel by drying and compressing the sponge material to a size and shape that is suitable for insertion into the cavity or vessel of interest. The thus dried and compressed device will remain in the compressed, or 'captive', state. Upon hydration, e.g. by fluids in the blood following insertion of the device into a bleeding cavity, the sponge will be released from its captive state and attempt to return to its relaxed state. In doing so the haemostatic fabric on the surface of the device can be pressed against the wall of the cavity or vessel with a haemostatically effective pressure.

In another embodiment a sponge device of the invention may be prepared for insertion into a cavity or vessel by the sustained compression of the mechanical means by a deployment means. Typically the type of sponge used in this embodiment does not set in a solid form when dry. A deployment means can be any device that is capable of providing sustained compression to the device and thus holding the device at a size and shape that is suitable for insertion into the cavity or vessel of interest. Usually the part of the deployment means intended for delivery of the device is itself of a size and shape that is suitable for insertion into the cavity or vessel of interest.

In a preferred embodiment the deployment means comprises a tube that is itself a size and shape suitable for insertion into the cavity or vessel of interest and which is capable of holding the compressed device at a size and shape that is suitable for insertion into the cavity or vessel of interest. Typically the tube will have an opening at one end, the distal end, to allow insertion and removal of the device. Preferably the tube will further comprise an extrusion means which is capable of forcing the device out of the tube through the opening at the distal end. The extrusion means typically comprises a piston and rod, which rod passes though an opening in the tube and can be operated from the proximal end of the tube. Thus upon insertion of the tube comprising a device of the invention into the cavity of vessel of interest, the device can be delivered into the cavity or vessel by maintaining the rod and piston, in a stationary position whilst withdrawing the tube from the cavity or vessel, preferably in the direction of the proximal end of the tube, or otherwise causing the piston to move from a proximal to a distal position within the tube, and thus causing the device to be extruded into the cavity or vessel. On release from the tube the sponge device can expand and press the haemostatic fabric on the surface of the device against the inner walls of the cavity at a haemostatically effective pressure.

The advantage of this method is that the material can be compressed into a circular cross section and remains soft during deployment (as distinct from the hydration activated dry sponge which is hard during deployment). The circular cross section means that a device can be provided with a relatively large volume but nevertheless with a relatively small width or diameter.

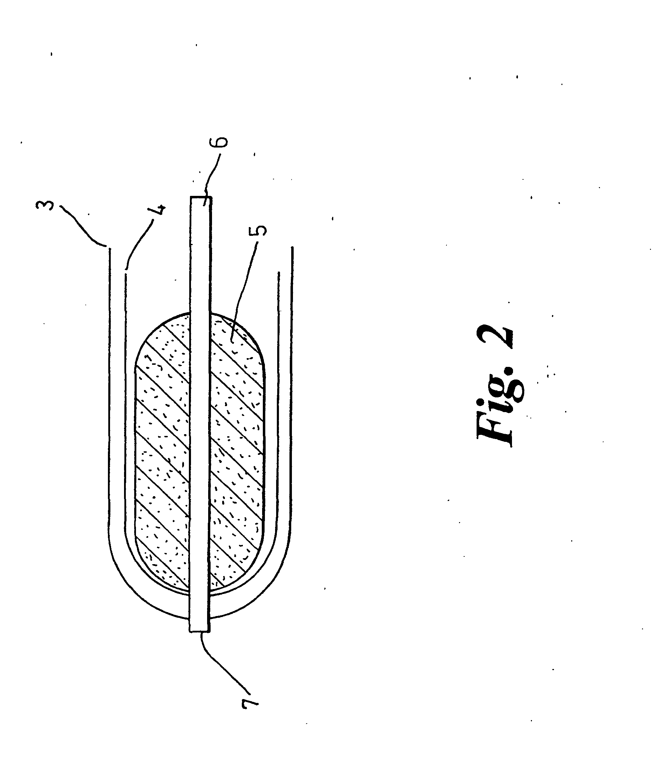

Fig 2 shows a diagrammatic cross section of one preferred device of the invention (for the sake of clarity the device is shown expanded in open air and not inside a cavity or vessel). A sponge (5) is surrounded by a friction-reducing layer (4) and a haemostatic fabric (3) after expansion. A central breathing tube (6) passes through the centre of the sponge. The lumen (7) of the breathing tube forms an airway. The haemostatic fabric (3) and the friction-reducing layer (4) are attached to the central breathing tube at the distal end of the sponge (5).

The term "stent" as used herein refers to a spring-type device that is designed to give an outward radial force against the wall of a cavity or vessel in which it is placed. Usually a stent for use in a stent device of the invention comprises a tubular open cage made out of a spring material. The stent may have a cylindrical configuration or may be barrel shaped or spherical as is appropriate to the shape of the cavity or vessel for which it is designed. Fig: 3 shows a typical cylindrical stent in the expanded position.

Stents for use in stent devices of the invention may be made from any suitable material and may be made by any method known in the art. Typically stents are made from stainless steel, nitinol or any other suitable elastic material. Nitinol is a binary alloy of nickel and titanium which has "super elastic" properties. It is well known as an excellent material for the manufacture of medical stents. Stents are usually manufactured from wire and then heat set, or cut from a tube using, e.g. laser cutting technology known in the art. Metallic stents are known in the art for use in medical applications and have been used to prevent elastic recoil after angioplasty in arteries. Arterial stents have been used in peripheral arteries as well as coronary arteries. Other stents have been used in the Trachea and the Oesophagus. These stents are usually made from metal such as stainless steel or nitinol, and are available in a range of mechanical configurations.

Stent devices of the invention may comprise a stent as a separate entity deployed inside the haemostatic fabric, or may be actually incorporated into the construction of the fabric.

Fabric covered stents are available which are used in two ways. First, the fabric of the covered stent can be used to isolate the wall of the cavity or vessel from the lumen in order to inhibit tissue growth into the lumen. Second, the covered stent can be used as a conduit for blood in the repair of aneurysms. Stent devices of the present invention may also be used to press a haemostatic fabric against the wall of a cavity or vessel containing a bleeding lesion.

In one embodiment a stent for use in a device of the present invention is in the form of a shaped cage-like structure covered with a haemostatic fabric. Thus the stent part and the fabric part of the device can be manufactured using known techniques.

In another embodiment a stent for use in a device of the present invention is in the form of a haemostatic fabric which incorporates, within the construction of the fabric, longitudinal and / or radial spring like components. Typically the haemostatic fabric remains predominantly on the outside of the device while the spring like elements are predominately on the inside of the device. The spring part(s) may be made of any suitable material but typically comprise or consist of a thermoplastic polymer, preferably polyamide (nylon) or polyester. The spring part(s) are usually in the form of monofilaments, which monofilaments have a diameter that gives the spring to give stability in the completed device. The spring(s) can then be incorporated into a basic haemostatic fabric tube. Fig: 4 shows a section of fabric with a spring or strut member (8) "laid in" to the knitted construction. These struts are shown horizontally, but vertical (longitudinal), or diagonal struts can be incorporated in a similar manner. After the fabric tube is made it can be shaped into a form that is suitable for insertion into the cavity or vessel of interest. This may be done by any method known in the art, for example, by holding the fabric over a suitably shaped form and subjecting the fabric to sufficient heat in order to "set" the shape. The temperature of the treatment is sufficient if the thermoplastic (spring strut) elements of the device take on a permanent set. Preferably the haemostatic agent in the haemostatic fabric will have a higher melting point than the thermoplastic spring strut elements and is not thermoplastic. Therefore, in a preferred embodiment the haemostatic fabric not affected by the heat shaping process and remains soft and absorbent. The reinforcing yarn within the fabric may be shaped by the heat setting process. However, usually the reinforcing yarn is too thin to have any mechanical affect on the shape of the device. The final shape of the device can be varied to adapt to different body cavities, vessels and other applications.

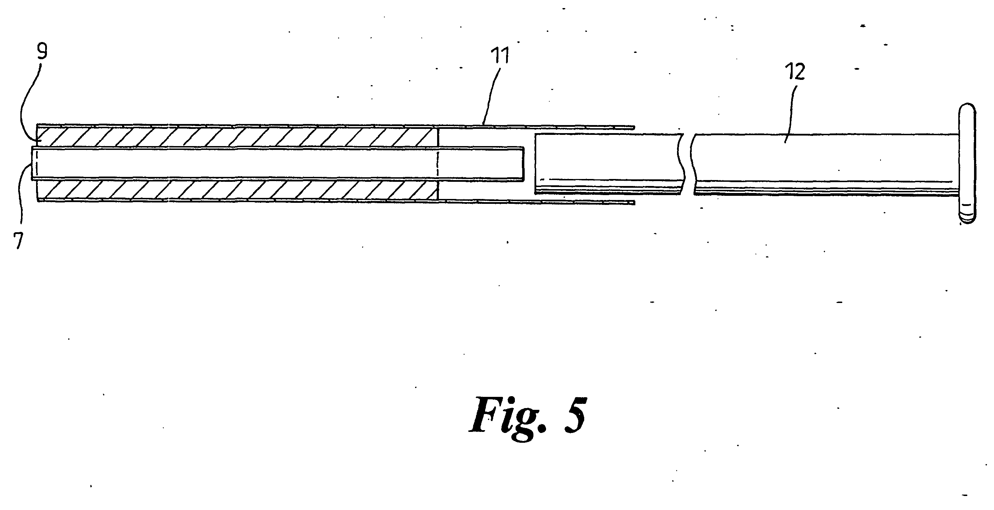

Stent devices of the invention may be compressed and held in a compressed state by a deployment means in the same manner as that discussed above in respect of sponge devices. Thus the present invention also provides a method for preparing devices of the invention for introduction into a body cavity or vessel by arranging the device in a manner that is suitable for insertion into the cavity or vessel of interest. Fig 5 shows a diagrammatic cross section of a typical deployment kit for deployment of a stent device of the invention. The stent, covered by the haemostatic fabric (9) is compressed and held in the compressed state by a tube (11). A piston (12) is provided for extruding the device from the tube. In the case of a dry sponge device, a friction-reducing layer would be placed between the sponge and the haemostatic fabric.

### Haemostatic Fabric

A haemostatic fabric is a fabric which comprises an agent that retards or arrests the flow of blood. The term "an agent that retards or arrests the flow of blood" includes any haemostatic agent that is capable of arresting, or stemming bleeding.

Examples of preferred haemostatic agents that retard or prevent bleeding include oxidised cellulose, such as Tabotamp™ sold by Johnson and Johnson, calcium alginate, gelatine or collagen. A particularly preferred agent is carboxymethylated cellulose (CMC) which is commercially available in various forms. It is used in many industries as a swelling agent due to its gelling properties when wetted. In this invention the CMC is made by converting a cellulosic precursor fabric into CMC to the preferred degree. The precursor fabric may also contain a reinforcing yarn as described in PCT/GB00/03586.

CMC gels upon contact with water, blood or body fluids, and swells to absorb such materials. CMC also facilitates blood clotting while absorbing any exude and is, therefore, haemostatic. In addition, CMC is hydroscopic so it does not readily dry into clotted blood, and therefore can be removed easily without causing re-bleeding. If it does dry, it can be easily re-gelled by wetting with water or saline solution.

In a preferred embodiment the haemostatic fabric comprises a composite fabric, which retains its structural integrity while absorbing a large quantity of fluid, and particularly to such a fabric useful for the control of bleeding.

The word "yarn" as used herein refers to an indefinite length of material suitable for weaving, knitting or braiding, typically comprised of one or more continuous strands of material or a multiplicity of relatively short length fibres spun into a fibre bundle of indefinite length or a combination of continuous strands and spun fibres.

The term "reinforcing" yarn refers to a yarn that has greater tensile strength in a wet phase than a gel-forming yarn with which it is combined.

"Gel-forming" materials or yarns, of the type generally referred to herein, typically soften to' form a gel or partially dissolve when brought into contact with a suitable liquid such as blood. Accordingly the composition of the haemostatic fabric, and thus the device, can be changed by its use. Gel-forming materials absorb liquid and will absorb many times their own weight. Preferably the gel-forming materials or yarns used in the invention comprise a haemostatic agent as described above because they tend to cause blood clotting while absorbing any exudate. Haemostatic, gel-forming materials, such as CMC, are particularly useful for medical purposes wherein the absorption of body fluids is important. Such materials are also used during surgery, or other medical procedures, as haemostatic agents and wound dressings.

The preferred composite fabrics used in the present invention typically comprise a reinforcing yarn woven, knitted or braided with a gel-forming yarn. Usually the reinforcing yarn is a relatively strong synthetic material, with which the gel-forming yarn is placed side by side during the weaving, knitting or braiding of the gel-forming and reinforcing yarns into a woven, knitted or braided fabric.

Alternatively, all or less than all of the yarn courses of the reinforcing yarn may be accompanied by gel-forming yarn. Alternatively also, still other yarn courses or picks of the woven or knitted fabric may comprise gel-forming yarn only, so long as the network of woven or knitted reinforcing yarn retains its structural integrity independent of the gel-forming yarn.

In general, it is preferred for present purposes to maximise the proportion of gel-forming yarn in the fabric and incorporate as little as possible of the reinforcing yarn, while still ensuring adequate strength in the fabric after the gel-forming yarns have gelled. As a practical matter, at least 5% (by weight) of reinforcing yarn is required but a larger proportion of reinforcing yarn may be used to yield a fabric of greater strength.

CMC may be made by the chemical conversion of a variety of cellulosic materials, such as viscose rayon, cotton, etc. One cellulosic yarn suitable for the present invention is a Lyocell yarn. It is available from a number of yarn spinners world wide and is a readily available commercial yarn. Lyocell is a solvent spun cellulose, produced from the natural cellulose in wood pulp by dissolution of the pulp in a solvent and then extruding the solution through a multiple-hole die, called a spinneret, to form a yarn comprised of a plurality of continuous strands. The solvent is vaporized in the process, leaving a continuous multi-filament yarn composed of pure cellulose.

The filaments in such a yarn may be chopped into staple form and spun into a yarn in a way similar to that used in processing cotton fibre.

Similar yarns may be used which are made from cotton or viscose rayon. Such yarns and the technology for their production are well known to those skilled in the art of textile technology.

In one embodiment of the present invention, such an unconverted cellulose yarn is readily woven, knit or braided into a precursor fabric, from which the fabric of the present invention is made by conversion of the cellulose to sodium carboxymethylcellulose or to oxidized cellulose, in accordance with known techniques. A useful reference to CMC production from cellulosic materials can be seen in the Journal of Applied Polymer Science Volume 17, pages 3375 - 3389 (1973).

In the conversion of cellulose to sodium carboxymethylcellulose, less than all of the cellulose building blocks may be converted to the sodium carboxymethylcellulose form and the degree of this conversion will dictate the degree to which a resultant CMC yarn will absorb water and form a gel therewith. This proportion is sometimes referred to as the degree of substitution or the conversion factor. While the present invention is not limited to sodium carboxymethylcellulose of any particular conversion factor, such materials with a degree of substitution of at least 0.1 and less than 0.9 are preferred in the fabric of the present invention. A more preferred material has a degree of substitution of between 0.20 and 0.35.

Oxidized cellulose, which is conventionally used in knitted form as a haemostatic agent during surgery, may also be used in the reinforced fabric of the present invention and may also be converted (oxidized) after cellulosic yarn is first woven, knit or braided into a precursor fabric.

Yet another haemostatic material, useful in the present invention, is calcium alginate, which is a material derived from seaweed, and, in matted fibre form, is also used as a wound dressing. Other fibrous polysaccharides, with similar chemistry and properties to CMC, may also be used.

Combinations of different gel-forming agents may be used within the scope of the present invention. Such combinations may be made by forming a yarn from different gel-forming or haemostatic fibres and/or by weaving, knitting or braiding combinations of different gel-forming yarns.

In the case where a precursor fabric is first formed with cellulose yarn, and the knitted, woven or braided cellulose yarn is then converted to gel-forming oxidized cellulose or sodium carboxymethylcellulose, the reinforcing yarn must be nonreactive with the reactants and the products of the process of converting the cellulosic material into the gel-forming, chemically modified form thereof.

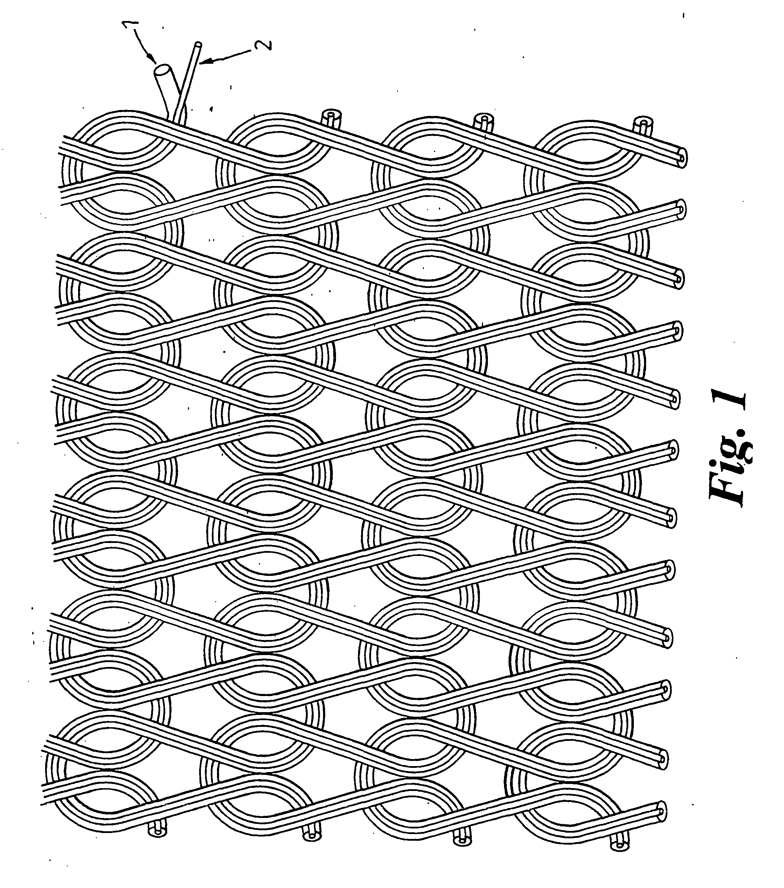

Referring to Figure 1 the step of weaving, knitting or braiding involves conventional methods, which are known. In accordance with the present invention, each of the multiple yarn end feeds to a weaving loom, knitting machine or braiding machine may comprise, in effect, two yarn ends, fed in parallel, one the gel-forming yarn (or a precursor yarn suitable for subsequent conversion to a gel-forming yarn), and one the reinforcing yarn. With a weft knit fabric constructed in this way as an example, the knit fabric product would include, as shown in Figure 1, a thin reinforcing yarn 2, combined in all yarn courses with a thicker (but weaker) yarn 1, which is either a gel-forming fibre or is convertible to a gel-forming yarn (i.e. a gel-forming yarn precursor).

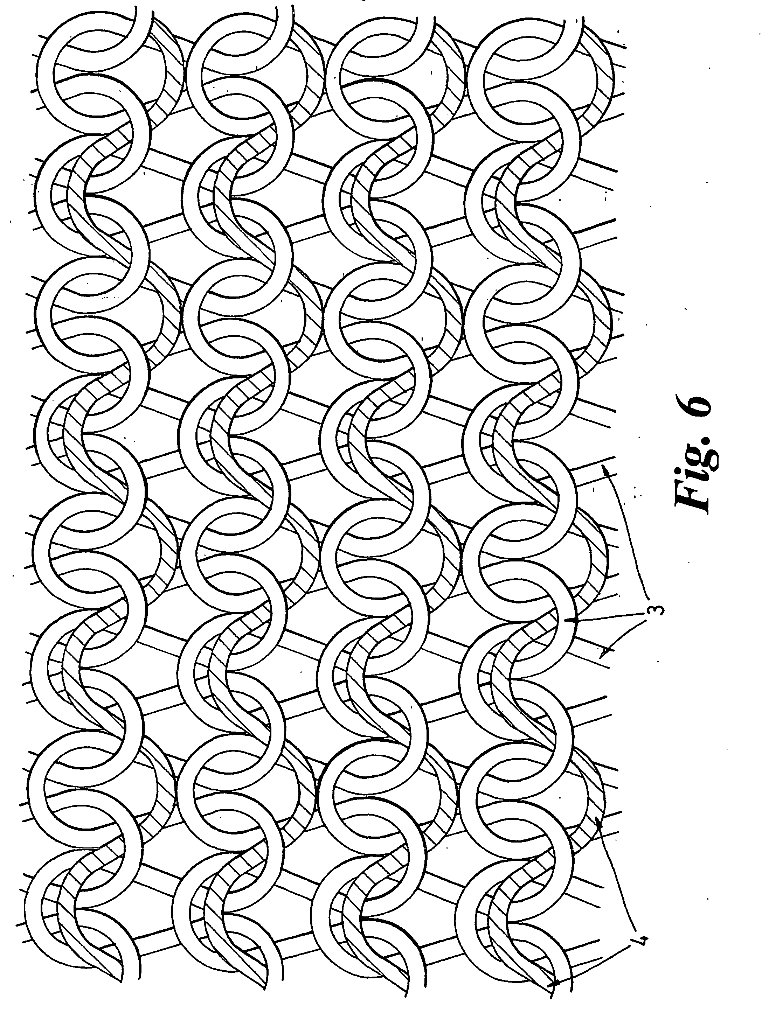

In such a structure, at least some of the gel-forming yarn courses may be omitted, depending on the relative degree of strength and absorptive capacity desired. Shown in Figure 6 is another knit fabric of the present invention. Reinforcing yarns 3 are knit so as to provide structural integrity to the fabric, while gel-forming (or precursor to gel-forming) yarns 4 are inlaid therewith. The inlaying of gel-forming yarns 4 is such that even if the gel-forming yarns are fully dissolved, the network of reinforcing yarns will maintain the structural integrity of the fabric.

Knit forms of the composite fabric of this invention have some inherent stretchability. In certain embodiments of the fabrics, such as those shown in Figures 1 and 2, still more stretchability may be provided. More specifically, the reinforcing yarn itself may be stretchable so that the fabric itself is more stretchable.

While the range of fabrics required for different applications is very wide, an exemplary fabric, made for use in a nasal haemostatic device, comprises a knit construction, as illustrated in Figure 1, knitted into a tubular form in accordance with well-known methods. In this exemplary fabric, a gel forming precursor yarn (12 tex lyocell, cotton or viscose spun yarn is knit together with a reinforcing yarn comprised of 17 decitex 3 filament nylon). The fabric structure is a plain weft, knitted in circular form with 36 needles. The loop length is 5 mm and the weight of the finished fabric is 1.6 grams per metre (wet relaxed and dried to normal moisture regain). The reinforcing yarn comprises about 12%, by weight, of this fabric before conversion of the Lyocell to CMC and about 11% after that conversion.

The conversion of the Lyocell in this exemplary fabric is accomplished by methods well known in the art.

While the nylon reinforcing yarn used in this embodiment would not be considered stretchable, the fabric structure itself is stretchable and deformable, that is it will expand in diameter at the expense of its length.

Apart from the composite fabric as described above, the present invention also includes the process of making a gel-forming or haemostatic structure, including a matted fibre or laid-in knit structure, as disclosed in WO 98/46818, by first forming the structure with gel-forming fibre precursors, such as cellulose fibre or yarn, and then converting the structure to the gel-forming state thereof, namely oxidized cellulose or CMC.

Still other composites and fabrics within the scope of this invention comprise a composite yarn, the structure of which includes both reinforcing fibres, such as nylon, and gel-forming fibres (or precursors thereof).

The most elementary method of combining two different fibres within one yarn is to simply spin the yarn from a mixture of the two fibres in staple form. However, this may lead to an overly weakened yarn once the gelling has taken place.

A preferred example of such a composite yarn is a core spun yarn, that is a yarn wherein staple fibres are spun around a preformed yarn. This preformed yarn may be another spun yarn, or, more commonly, a continuous filament yarn. This preformed yarn may comprise a reinforcing material, such as nylon. Gel-forming, or precursors of gel-forming, fibres comprise a second component of the final yarn product. The gel-forming fibres therein (converted from precursor materials either prior to or after spinning) provide absorptive and haemostatic capacity to the yarn and the reinforcing fibres or central filament of the preformed yarn provide strength. Such a yarn may be woven, knit or otherwise incorporated into a fabric or other structure, wherein fluid or blood absorption are important.

In one embodiment a preferred haemostatic fabric is a weft knitted fabric fabricated with two yarns as shown in Fig. 1. The main yarn (1) is spun from fibres of a gellable haemostatic material comprising a gellable haemostatic agent such as CMC. A non-gelling reinforcing yarn (2) serves to maintain the integrity of the fabric structure after the gelling has taken place.

In further embodiments the haemostatic device may comprise a filled haemostatic fabric bag. The filled bag may resemble a tampon and, for convenience, may be provided with a string or other withdrawal means for withdrawing the device from a cavity or vessel.

The bag may be formed from any suitable fabric. Preferably the bag is formed from a fabric which comprises CMC.

In use, the filling serves as a mechanical means for outwardly expanding the fabric against the inner wall of a cavity or vessel. The filling material can be formed from any suitable material. Preferably the filling material is a fabric which comprises CMC.

The inventor has found that the fabric which forms the bag can be non-haemostatic provided that the filling material is haemostatic. Hence, the bag and/or filling material may be haemostatic

A variety of combinations could be used.
- Haemostatic fabric with non haemostatic filler
- Haemostatic filler with non haemostatic fabric
- Filler and fabric haemostatic.

There must be at least one component that is haemostatic. Either the fabric, the filler or both.

In some cases it is necessary to have a device with the maximum absorbency where a high amount of wound exudate is encountered. In this case the filler may be a high absorbency material. Such filler may be haemostatic or non-haemostatic. It should be noted that CMC material has the property of very high absorbency as well as the property of being a powerful haemostatic agent.
The filler material may be loose textile fibre in the form of wadding, or rolled up or packed fabric. Indeed the filler can be made of the same material as the outer fabric, either in its haemostatic (CMC) form or its precursor non-haemostatic cellulosic form.

In other cases the total amount of absorbent material must be kept to a minimum to reduce the risk of toxic shock syndrome. An example of this is a possible use for the device as a haemostatic device used after surgery of the vagina. It is well known that large amounts of blood soaked up in an absorbent material can give rise to bacterial growth which leads to toxic shock syndrome. (TSS has been found to be especially likely to be caused by tampons soaked in menstrual blood.) There is, therefore, a need for a device which uses a haemostatic fabric with a non haemostatic filler, when the filler is non absorbent (waterproof).

Alternatively, the filling may take the form of a sponge.

The sponge may be pre-formed into a suitable shape in order to conform to a particular body cavity.

Alternatively, the filling may be in the form of a resilient member such as a plastic tube, or substantially tubular member.

Alternatively, the filling may be in the form of a gas filled balloon or air pillow. In a permanently air filled balloon, the elastic resilience of the air would allow the balloon (and haemostatic fabric cover), to be compressed into a smaller profile and contained within a deployment or delivery means.

The pressure of the air within the balloon is predetermined at the correct level to give the correct tamponade pressure when inside the body cavity.

Such an air pillow filling or resilient plastic tube fillings have the advantage of providing a haemostatic device for a large cavity and with the minimum of absorbent material. A number of relatively small such units may be used to pack a body cavity after surgery or trauma.

The air filled pillow may be pre-formed into a suitable shape in order to conform to a particular body cavity. For example a substantially triangular shape is suitable for some sinus cavities in the head.

Sponge, wadding or fabric, filled devices of suitable size can be used for the so called "FESS" (Functional Endoscopic Sinus Surgery) procedure in nasal sinus surgery.

In any of the embodiments of the invention a biocidal agent may be provided to reduce the risk of toxic shock. Skilled persons will appreciate that a range of biocidal agents are available and that such agents could be provided by using them to impregnate or coat elements of the devices of the invention.

### Friction-Reducing Layer

Experiments have shown frictional forces between the inner surface of the haemostatic fabric and the outer surface of the surface of the mechanical means for expansion can cause drag during expansion leading to a reduction of the expansion. This is especially true when using a sponge as the expansion means. This can be addressed by lining the haemostatic fabric with a friction-reducing layer. Thus in a preferred embodiment, devices of the invention comprise a layer of material between the mechanical expansion means and the haemostatic fabric, which material is capable of reducing friction between the outer surface of the mechanical expansion means and the inner surface of the haemostatic fabric. Preferably the reduction in friction allows the fabric to readily expand.

Thus devices of the invention may not comprise a friction-reducing layer, in which case the layer adjacent to the outer surface of the mechanical expanding means may be the haemostatic material. However, in a preferred embodiment the outer layer of the mechanical expansion means and the inner surface of the friction-reducing layer are separated by a friction-reducing layer and thus in preferred devices of the invention the layer adjacent to the outer surface of the mechanical expanding means is the friction reducing layer. Typically the friction experienced between the outside surface of the mechanical expanding means and the inner surface of the adjacent layer, where the friction experienced in devices with no friction-reducing layer is referred to as 100%, is at most 70%, yet more typically at most 50%, preferably at most 30%, more preferably at most 20%, yet more preferably at most 10%, even more preferably at most 5% when the adjacent layer is a friction reducing layer. The preferred friction-reducing material is polytetrafluoroethane (PTFE).

Typically the friction-reducing layer is thin. The term "thin" as used in the context of the friction reducing layer refers to the thickness of the layer and is usually less than the thickness of the haemostatic fabric, typically at most 75%, 50% or 25% of the thickness of the haemostatic fabric. In a preferred embodiment devices of the invention which have a friction-reducing layer have a sponge as the mechanical expansion means. Wherein the mechanical expansion means is a sponge which is released to expand by hydration, it is preferable that the friction-reducing layer does not prevent the fluid or fluids of the body cavity or vessel from impregnating the sponge. Thus in a preferred embodiment the friction-reducing layer does not form a continuous layer around the sponge. The friction-reducing layer may comprise one or more pores or perforations that allow the sponge to be wetted by the fluid or fluids of the body cavity or vessel.

### Method For Stopping Bleeding / Use Of Device To Stop Bleeding

A device of the invention can be used in a method of stopping bleeding from a lesion in a body cavity or vessel. Typically the device is first prepared by arranging the device in a manner that is suitable for insertion into the cavity or vessel of interest.

The device is then inserted into the cavity or vessel of choice. Where the device is a provided in a deployment means, the device is then extruded from the deployment means. The device is then allowed to expand within the cavity or vessel until the outer surface of the device contacts the inner wall of the cavity or vessel. The haemostatic fabric is thus applied to the cavity or vessel wall The location of the device is then maintained for a time sufficient to bring about haemostasis.

Once haemostasis has occurred the device may optionally be removed from the cavity or vessel. Preferably the device is removed without causing an increase in the rate of bleeding. Devices of the invention may be easily removed since the mechanical expansion means remains compressible and the gellable haemostatic agent on the surface of the device turns into a gel, thus providing a 'non-stick' barrier between the device and lesion site which ensures that removal of the device does not cause the formed blood clot to be substantially disturbed. If the clot and gell has been allowed to dry, it can easily be softened by wetting the fabric with saline solution by means of a syringe, thus causing the fabric to re-gell.

In a preferred embodiment, a device of the invention is used in a method of packing a cavity after surgery to control bleeding. Accordingly the use of a device of the invention can enhance healing within a cavity. Typically, the cavity is a nasal cavity or sinus within the head. Other applications are, inter alia, the ear, and the vagina.

## Claims

1. A haemostatic device suitable for use in a body cavity or vessel having an inner wall, comprising a haemostatic fabric (3,9) and a mechanical means for outwardly expanding the fabric against the inner wall of the cavity or vessel **characterised in that** the haemostatic fabric is a composite knitted, woven or braided fabric comprising a combination of:
gel-forming yarn (1) or gel-forming yarn precursor (1), said gel-forming yarn (1) or gel-forming yarn precursor (1) being woven, knitted or braided with a reinforcing yarn (2), wherein the knitting, weaving or braiding of such reinforcing yarn (2) forms a network capable of providing physical integrity to said fabric independent of said gel-forming yarn (1) or gel-forming yarn precursor (1), and **in that** the mechanical means is selected from:
(i) a sponge (5);
(ii) a stent;
(iii) a gas-filled balloon or air-filled pillow;
(iv) an elastic tube or tubular member;
(v) a thermoplastic spring or strut (8), which thermoplastic spring or strut (8) forms a part of the fabric construction; or
(vi) the device comprises a fabric bag filled with filling material,
wherein the filling material constitutes said mechanical means.

2. A device according to Claim 1 wherein the haemostatic fabric (3,9) comprises a haemostatic yarn.

3. A device according to Claim 1 or 2 wherein the haemostatic fabric (3,9) comprises a gel-forming haemostatic yarn (1).

4. A device according to claim 1 wherein said gel-forming yarn (1) is comprised of sodium carboxymethylcellulose.

5. A device according to claim 1 wherein said gel-forming yarn (1) is selected from the group consisting of sodium carboxymethylcullulose, oxidized cellulose, and calcium alginate.

6. A device according to any preceding claim wherein said reinforcing filament is a nylon continuous mono or multifilament yarn.

7. A device according to claim 1 wherein the haemostatic fabric (3,9) is a composite fabric comprising a woven, knitted or braided combination of:
one or more yarns capable of gelling upon contact with liquid (1), and
one or more reinforcing yarns (2),
wherein said reinforcing yarn (2) has greater tensile strength than said gelling yarn (1) in a wet phase, and
wherein said fabric (3,9) is highly absorbent to blood and body fluids and
wherein the woven, knit or braided network of said reinforcing yarn (2) is capable of providing structural integrity to said fabric independent of said yarn capable of gelling upon contact with liquid (1).

8. A device according to any one of the preceding claims further comprising a friction-reducing layer (4) which reduces the friction between the haemostatic fabric (3,9) and the surface of the mechanical means during outwards expansion of the device.

9. A device according to claim 8 wherein friction-reducing layer (4) comprises polytetrafluoroethane.

10. A device according to any one of the preceding claims wherein the sponge (5) comprises polyvinyl alcohol (PVA) polymer or a functional equivalent thereof.

11. A device according to any one of the preceding claims wherein the friction-reducing layer (4) comprises perforations that allow the sponge to be wetted.

12. A device according to any one of the preceding claims wherein the sponge (5) can expand from a compressed captive position in the dry state.

13. A device according to any one of the preceding claims wherein the stent comprises nitinol, stainless steel or a synthetic polymer.

14. A device according to any one of the preceding claims wherein the synthetic polymer is nylon or polyester.

15. A device according to any one of the preceding claims which further comprises a deployment means.

16. A device according to claim 15 wherein the deployment means comprises a tube (11) and a piston (12) and rod, which piston (12) is movable relative to the length of the tube (11) and is arranged so that movement of the rod and piston (12) relative to the length of the tube (11) in the direction of the distal end of the tube (11) causes the haemostatic device to be extruded from the distal end of the tube (11).

17. A device as claimed in any one of the preceding claims wherein the filling material is selected from a haemostatic fabric (3,9), a non-haemostatic fabric, a haemostatic textile fibre wadding, a non-haemostatic textile wadding, a sponge (5), a resilient tube, or a gas filled balloon.

18. A device as claimed in any one of the preceding claims wherein the fabric bag comprises a haemostatic fabric.

19. A device as claimed in any preceding claim wherein the device is provided with a release means for withdrawing the device from a cavity or vessel.

20. A device as claimed in any preceding claim wherein the device is provided with a biocidal agent.

21. A haemostatic device according to any preceding claim wherein the filler is made of the same material as the outer fabric.

## Patentansprüche

1. Blut stillende Vorrichtung, die für die Verwendung bei einem Körperhohlraum oder Gefäß geeignet ist, der bzw. das eine innere Wand hat, wobei sie einen Blut stillenden Stoff (3, 9) und eine mechanische Einrichtung zum Ausdehnen des Stoffes nach außen gegen die innere Wand des Hohlraums oder des Gefäßes aufweist, **dadurch gekennzeichnet, dass** der Blut stillende Stoff ein zusammengesetzter bzw. gemischter gewirkter bzw. gestrickter, gewebter oder geflochtener bzw. umsponnener Stoff ist, der folgende Zusammensetzung aufweist:
Gel bildendes Garn bzw. Faden (1) oder Gel bildendes Garn- bzw. Fadenvorprodukt (1), wobei das Gel bildende Garn bzw. Faden (1) oder Gel bildende Garn- bzw. Fadenvorprodukt (1) gewebt, gewirkt bzw. gestrickt oder mit einem Verstärkungsgarn bzw. - faden (2) geflochten bzw. umsponnen ist, wobei das Wirken bzw. Stricken, Weben oder Flechten bzw. Umspinnen eines derartigen Verstärkungsgarnes bzw. -fadens (2) ein Netz bzw. Geflecht bildet, das imstande ist, physische Reinheit bzw. Unversehrtheit bei dem Stoff vorzusehen, unabhängig von dem Gel bildenden Garn bzw. Faden (1) oder dem Gel bildenden Garn- bzw. Fadenvorprodukt (1), und dass die mechanische Einrichtung aus Folgendem ausgewählt wird:
(i) einem Schwamm (5);
(ii) einem Stent;
(iii) einem gasgefüllten Ballon oder luftgefüllten Kissen;
(iv) einem elastischen Rohr bzw. Schlauch oder rohrförmigen Glied;
(v) einer thermoplastischen Feder oder Strebe (8), wobei die thermoplastische Feder oder Strebe (8) einen Teil des Stoffaufbaus bildet; oder
(vi) die Vorrichtung weist einen Stoffsack bzw. -beutel auf, der mit Füllmaterial gefüllt ist, wobei das Füllmaterial die mechanische Einrichtung bildet.

2. Vorrichtung gemäß Anspruch 1, wobei der Blut stillende Stoff (3, 9) ein Blut stillendes Garn bzw. Faden aufweist.

3. Vorrichtung gemäß Anspruch 1 oder 2, wobei der Blut stillende Stoff (3, 9) ein Gel bildendes Blut stillendes Garn bzw. Faden (1) aufweist.

4. Vorrichtung gemäß Anspruch 1, wobei das Gel bildende Garn bzw. Faden (1) von Natrium-Carboxymethylcellulose eingeschlossen bzw. umfasst ist.

5. Vorrichtung gemäß Anspruch 1, wobei das Gel bildende Garn bzw. Faden (1) aus der Gruppe ausgewählt wird, die aus Natrium-Carboxymethylcellulose, oxidierter Cellulose und Calciumalginat besteht.

6. Vorrichtung gemäß irgendeinem vorhergehenden Anspruch, wobei das Verstärkungsfilament bzw. -faser ein kontinuierliches Nylon-Mono- oder Multifilamentgarn bzw. -faden ist.

7. Vorrichtung gemäß Anspruch 1, wobei der Blut stillende Stoff (3, 9) ein zusammengesetzter bzw. gemischter Stoff ist, der eine gewebte, gewirkte bzw. gestrickte oder geflochtene bzw. umsponnene Zusammensetzung von Folgendem aufweist:
ein oder mehr Garne bzw. Fäden, die bei Kontakt mit Flüssigkeit (1) zur Gelbildung imstande sind, und
ein oder mehr Verstärkungsgarne bzw. -fäden (2),
wobei das Verstärkungsgarn bzw. -faden (2) größere Zugfestigkeit bzw. Zerreißfestigkeit hat als das Gelbildungsgarn bzw. -faden (1) in einer feuchten Phase bzw. Stadium, und
wobei der Stoff (3, 9) hochabsorbierend bzw. hoch aufsaugend bei Blut und Körperfluiden ist, und
wobei das gewebte, gewirkte bzw. gestrickte oder geflochtene bzw. umsponnene Netz bzw. Geflecht des Verstärkungsgarns bzw. -fadens (2) imstande ist, eine strukturelle Reinheit bzw. Unversehrtheit bei dem Stoff vorzusehen, unabhängig von dem Garn bzw. Faden, der bei Kontakt mit Flüssigkeit (1) zur Gelbildung imstande ist.

8. Vorrichtung gemäß irgendeinem der vorhergehenden Ansprüche, die ferner eine reibungsmindernde Schicht (4) aufweist, welche die Reibung zwischen dem Blut stillenden Stoff (3, 9) und der Oberfläche der mechanischen Einrichtung während der Ausdehnung der Vorrichtung nach außen verringert.

9. Vorrichtung gemäß Anspruch 8, wobei die reibungsmindernde Schicht (4) Polytetrafluorethan aufweist.

10. Vorrichtung gemäß irgendeinem der vorhergehenden Ansprüche, wobei der Schwamm (5) Polyvinylalkohol (PVA) -Polymer oder ein zweckmäßiges Äquivalent davon aufweist.

11. Vorrichtung gemäß irgendeinem der vorhergehenden Ansprüche, wobei die reibungsmindernde Schicht (4) Perforationen aufweist, die es dem Schwamm ermöglichen, benetzt bzw. befeuchtet zu werden.

12. Vorrichtung gemäß irgendeinem der vorhergehenden Ansprüche, wobei sich der Schwamm (5) aus einer zusammengedrückt festgehaltenen bzw. gefangenen Position in dem trockenen Zustand ausdehnen kann.

13. Vorrichtung gemäß irgendeinem der vorhergehenden Ansprüche, wobei der Stent Nitinol, rostfreien Stahl oder ein synthetisches Polymer aufweist.

14. Vorrichtung gemäß irgendeinem der vorhergehenden Ansprüche, wobei das synthetische Polymer Nylon oder Polyester ist.

15. Vorrichtung gemäß irgendeinem der vorhergehenden Ansprüche, welche ferner eine Einsatzeinrichtung aufweist.

16. Vorrichtung gemäß Anspruch 15, wobei die Einsatzeinrichtung ein Rohr (11) und einen Kolben (12) und eine Stange aufweist, wobei der Kolben (12) relativ zu der Länge des Rohres (11) bewegbar ist und so angeordnet ist, dass Bewegung der Stange und des Kolbens (12) relativ zu der Länge des Rohres (11) in der Richtung des distalen Endes des Rohres (11) die Blut stillende Vorrichtung veranlasst, aus dem distalen Ende des Rohres (11) ausgestoßen zu werden.

17. Vorrichtung wie in irgendeinem der vorhergehenden Ansprüche beansprucht, wobei das Füllmaterial aus einem Blut stillenden Stoff (3, 9), einem nicht Blut stillenden Stoff, einer Blut stillenden Textilfasereinlage bzw. -füllung, einer nicht Blut stillenden Textileinlage bzw. -füllung, einem Schwamm (5), einem elastischen Rohr bzw. Schlauch oder einem gasgefüllten Ballon ausgewählt ist.

18. Vorrichtung wie in irgendeinem der vorhergehenden Ansprüche beansprucht, wobei der Stoffsack bzw. -beutel einen Blut stillenden Stoff aufweist.

19. Vorrichtung wie in irgendeinem vorhergehenden Anspruch beansprucht, wobei die Vorrichtung mit einer Freigabe- bzw. Löseeinrichtung zum Entfernen bzw. Herausziehen der Vorrichtung aus einem Hohlraum oder Gefäß versehen ist.

20. Vorrichtung wie in irgendeinem vorhergehenden Anspruch beansprucht, wobei die Vorrichtung mit einem bioziden Mittel versehen ist.

21. Blut stillende Vorrichtung gemäß irgendeinem vorhergehenden Anspruch, wobei der Füllstoff aus demselben Material wie der äußere Stoff hergestellt ist.

## Revendications

1. Dispositif hémostatique approprié pour être utilisé dans une cavité du corps ou un vaisseau ayant une paroi interne, comprenant un tissu hémostatique (3, 9) et un moyen mécanique pour dilater vers l'extérieur le tissu contre la paroi interne de la cavité ou vaisseau, **caractérisé en ce que** le tissu hémostatique est un tissu composite, tricoté, tissé ou tressé comprenant une combinaison de :
un fil (1) formant gel ou un précurseur de fil (1) formant gel, ledit fil (1) formant gel ou le précurseur de fil (1) formant gel étant tissé, tricoté ou tressé avec un fil de renforcement (2), dans lequel le tricotage, le tissage ou le tressage de ce fil de renforcement (2) forme un réseau capable de fournir une intégrité physique audit tissu indépendamment dudit fil (1) formant gel ou dudit précurseur de fil (1) formant gel et **en ce que** le moyen mécanique est choisi parmi :
(i) une éponge (5) ;
(ii) un écarteur ;
(iii) un ballon rempli de gaz ou un coussin rempli d'air ;
(iv) un tube élastique ou un élément tubulaire ;
(v) un élément élastique thermoplastique ou entretoise (8), lequel élément élastique thermoplastique ou entretoise (8) forme une partie de la construction du tissu ou
(vi) le dispositif comprend un sac de tissu rempli avec un matériau de remplissage, dans lequel le matériau de remplissage constitue ledit moyen mécanique.

2. Dispositif selon la revendication 1, dans lequel le tissu hémostatique (3, 9) comprend un fil hémostatique.

3. Dispositif selon la revendication 1 ou 2, dans lequel le tissu hémostatique (3, 9) comprend un fil hémostatique (1) formant gel.

4. Dispositif selon la revendication 1, dans lequel ledit fil (1) formant gel est constitué de carboxyméthylcellulose sodique.

5. Dispositif selon la revendication 1, dans lequel ledit fil (1) formant gel est choisi dans le groupe comprenant la carboxyméthylcellulose sodique, la cellulose oxydée et l'alginate de calcium.

6. Dispositif selon l'une quelconque des revendications précédentes, dans lequel ledit filament de renforcement est un fil mono ou multifilament continu de nylon.

7. Dispositif selon la revendication 1, dans lequel le tissu hémostatique (3, 9) est un tissu composite comprenant une combinaison tissée, tricotée ou tressée de :
un ou plusieurs fils capables de gélifier au contact d'un liquide (1), et
un ou plusieurs fils de renforcement (2),
dans lequel ledit fil de renforcement (2) a une résistance à la traction supérieure à celle dudit fil (1) gélifiant dans une phase humide, et
dans lequel ledit tissu (3, 9) est très absorbant pour le sang et les fluides corporels et
dans lequel le réseau tissé, tricoté ou tressé dudit fil de renforcement (2) est capable de fournir une intégrité de structure audit tissu indépendamment dudit fil capable de gélifier au contact avec le liquide (1).

8. Dispositif selon l'une quelconque des revendications précédentes, comprenant, en outre, une couche (4) de réduction du frottement qui réduit le frottement entre le tissu hémostatique (3, 9) et la surface du moyen mécanique pendant la dilatation vers l'extérieur du dispositif.

9. Dispositif selon la revendication 8, dans lequel la couche de réduction du frottement (4) comprend du polytétrafluoroéthane.

10. Dispositif selon l'une quelconque des revendications précédentes, dans lequel l'éponge (5) comprend un polymère d'alcool polyvinylique (PVA) ou un équivalent fonctionnel de celui-ci.

11. Dispositif selon l'une quelconque des revendications précédentes, dans lequel la couche de réduction du frottement (4) comprend des perforations qui permettent à l'éponge d'être mouillée.

12. Dispositif selon l'une quelconque des revendications précédentes, dans lequel l'éponge (5) peut se dilater à partir d'une position captive comprimée dans l'état sec.

13. Dispositif selon l'une quelconque des revendications précédentes, dans lequel l'écarteur comprend du nitinol, de l'acier inoxydable ou un polymère synthétique.

14. Dispositif selon l'une quelconque des revendications précédentes, dans lequel le polymère synthétique est du nylon ou du polyester.

15. Dispositif selon l'une quelconque des revendications précédentes, qui comprend, en outre, un moyen de déploiement.

16. Dispositif selon la revendication 15, dans lequel le moyen de déploiement comprend un tube (11) et un piston (12) et une tige, lequel piston (12) est mobile par rapport à la longueur du tube (11) et est agencé afin que le mouvement de la tige et du piston (12) par rapport à la longueur du tube (11) dans la direction de l'extrémité distale du tube (11) provoque que le dispositif hémostatique soit extrudé à partir de l'extrémité distale du tube (11).

17. Dispositif selon l'une quelconque des revendications précédentes, dans lequel le matériau de remplissage est choisi parmi un tissu hémostatique (3, 9), un tissu non hémostatique, un rembourrage de fibre textile hémostatique, un rembourrage textile non hémostatique, une éponge (5), un tube résilient ou un ballon rempli de gaz.

18. Dispositif selon l'une quelconque des revendications précédentes, dans lequel le sac de tissu comprend un tissu hémostatique.

19. Dispositif selon l'une quelconque des revendications précédentes, dans lequel le dispositif est muni d'un moyen de libération pour retirer le dispositif d'une cavité ou d'un vaisseau.

20. Dispositif selon l'une quelconque des revendications précédentes, dans lequel le dispositif est muni d'un agent biocide.

21. Dispositif hémostatique selon l'une quelconque des revendications précédentes, dans lequel la charge est réalisée en le même matériau que le tissu extérieur.
